(19) **Europäisches Patentamt / European Patent Office / Office européen des brevets**

(11) **EP 4 427 768 A1**

(12) # EUROPEAN PATENT APPLICATION
published in accordance with Art. 153(4) EPC

(43) Date of publication:
**11.09.2024 Bulletin 2024/37**

(21) Application number: **22887054.9**

(22) Date of filing: **26.10.2022**

(51) International Patent Classification (IPC):
*A61M 1/36* (2006.01)  *B01D 15/00* (2006.01)
*B01J 20/22* (2006.01)  *B01J 20/26* (2006.01)
*B01J 20/28* (2006.01)

(52) Cooperative Patent Classification (CPC):
**A61M 1/36; B01D 15/00; B01J 20/22; B01J 20/26; B01J 20/28**

(86) International application number:
**PCT/JP2022/039889**

(87) International publication number:
**WO 2023/074729 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.11.2021 JP 2021178488**
**01.11.2021 JP 2021178489**

(71) Applicant: **Toray Industries, Inc.**
**Tokyo 103-8666 (JP)**

(72) Inventors:
• **YAMASHITA, Mariko**
**Otsu-shi, Shiga 520-8558 (JP)**
• **HAN, Aishan**
**Otsu-shi, Shiga 520-8558 (JP)**
• **SAKAGUCHI, Hirokazu**
**Otsu-shi, Shiga 520-8558 (JP)**
• **UENO, Yoshiyuki**
**Otsu-shi, Shiga 520-8558 (JP)**

(74) Representative: **Kador & Partner Part mbB**
**Corneliusstraße 15**
**80469 München (DE)**

(54) **CELL ADSORPTION MATERIAL AND CELL ADSORPTION COLUMN**

(57)    An objective of the present invention is to provide a cell adsorption material that adsorbs cells, such as LAP positive immune cells including LAP positive T-cells and LAP positive platelets, with high efficiency. A cell adsorption material comprises fibers or particles of a water-insoluble carrier to which at least one nitrogen-containing compound is bound. The nitrogen-containing compound is selected from the group consisting of polyamines and aliphatic amines. The fibers or the particles have a region A and a region B, the region A being a region within 1.0 $\mu$m from an outermost surface of a cross-sectional surface of the fibers or the particles, the region B being a region within 0.5 $\mu$m radius from a center of gravity of the cross-sectional surface of the fibers or the particles, a spectral intensity of $^{26}CN^-$ satisfies formulae (1) and (2) below when a spectral intensity of $^{25}C_2H^-$ in each of the regions measured by Time of Flight Secondary Ion Mass Spectrometry is set to be 1:

$$0.5 \leq \text{the spectral intensity of } ^{26}CN^- \text{ in the region A} \leq 3.0 \ \dots \text{ Formula (1);}$$

$$3.0 \leq \text{the spectral intensity of } ^{26}CN^- \text{ in the region A/the spectral intensity of } ^{26}CN^- \text{ in the region B} \leq 20.0 \quad \text{Formula (2).}$$

EP 4 427 768 A1

# Fig. 1

**Description**

Technical Field

The present invention relates to a cell adsorption material and a cell adsorption column for LAP positive immune cells or the like.

Background Art

It has been becoming clear that cancers closely relate to immunity. In recent years, it has been reported that immunosuppressive blood components rise in many advanced cancers. One of such blood components is a leukocyte, which is classified into a lymphocyte, a granulocyte, or a monocyte. Each of the leukocytes is further subdivided, and for example, the lymphocyte is classified into a T-cell, a B-cell, a natural killer cell, and the like.

Cancer cells are known to have a mechanism that induces immunosuppressive T-cells to escape from an immune mechanism by producing immunosuppressive substances typified by a Transforming growth factor-$\beta$ (hereinafter referred to as "TGF-$\beta$"). TGF-$\beta$ is produced as LAP-TGF-$\beta$ complexes with no physiological activity associated with the Latency Associated Peptide (hereinafter referred to as "LAP") with molecular weight of 75,000 by non-covalent bonds. When LAP is cleaved by proteolytic enzymes and cell-adhesion molecules, TGF-$\beta$ can bond to TGF-$\beta$ receptors of various kinds of cells so as to exhibit a physiological function.

LAP-TGF-$\beta$ complexes are known to bond, not only to cancer cells, but also to cell membranes of a part of immune cells. This immune cell to which the LAP-TGF-$\beta$ complexes bond is referred to as a LAP positive immune cell and contributes to the escape of cancer cells from the immune mechanism. In particular, LAP positive T-cells, one of the LAP positive immune cells, have drawn attention as a supply source of TGF-$\beta$ for cancer cells, and there has been developed adsorbing materials for removing these.

Meanwhile, platelets are also known as one of the supply sources of TGF-$\beta$. Therefore, if the LAP positive immune cells as a combination of the LAP positive T-cells and the LAP positive platelets, which are the supply sources of the immunosuppressive substance TGF-$\beta$, can be removed at the same time, further enhancement in therapeutic effect for cancers is expected.

While as a method for treating cancers, there have been developed drugs that inhibit a transmission of immunosuppressive signal transmitted from cancer cells, such as immune checkpoint antibodies, cases of contractions of autoimmune diseases due to side effects of the drugs are also recognized.

In order to improve immune functions while reducing the side effects, cell therapies that eliminate cancer cells by leukocytes of a patient himself/herself have been performed. A representative method is a dendritic cell infusion therapy that treats cancers by returning dendritic cells of a patient that have been caused to extracorporeally ingest cancer antigens to the patient to induce cancer-specific killer T-cells. However, at present, this therapeutic method is regarded to have insufficient therapeutic effects. One of the reasons is presumed to be an enhanced immunosuppressive system.

Meanwhile, if the LAP positive immune cells as the supply source of TGF-$\beta$ can be removed, the immune response against the cancer cells is maintained, and thus, it is expected that extinction of the cancer cells and suppression of cancer development are possible.

Patent Literature 1 discloses a leukocyte removal filter formed of a nonwoven fabric having a fiber diameter of less than 3 $\mu$m and a bulk density of more than 0.15 g/cm$^3$ and 0.50 g/cm$^3$ or less as a material that removes leukocytes.

Patent Literature 2 discloses a cell adsorption column formed by being filled with an adsorbent including fibers with a fiber diameter of 0.5 $\mu$m or more and 10 $\mu$m or less and having a surface area of 0.5 m$^2$ or more and less than 10 m$^2$, and characterized by having an adsorbent-filled volume of 100 mL or less.

Patent Literature 3 discloses an adsorption material for immunosuppressive leukocytes with arithmetic roughness of a fiber surface of 0.1 $\mu$m or more and 3.0 $\mu$m or less.

Citation List

Patent Literature

Patent Literature 1: JP 60-193468 A
Patent Literature 2: WO2008/038785
Patent Literature 3: WO2019/049962

Summary of Invention

Technical Problem

However, the filter in Patent Literature 1 removes leukocytes by filtering, and therefore, there is a problem that all kinds of leukocytes are removed. The adsorption material in Patent Literature 2 adsorbs cells using phagocytic activities of granulocytes and monocytes, and therefore, there is a possibility that it is difficult to remove LAP positive T-cells. Although Patent Literature 3 discloses the adsorption material that removes immunosuppressive leukocytes by controlling surface roughness, it does not disclose removal of LAP positive platelets.

For the above-mentioned reasons, it is desired to develop materials that can adsorb and remove immune cells to which LAP-TGF-$\beta$ complexes bond.

Therefore, an object of the present invention is to provide a cell adsorption material that adsorbs cells, such as LAP positive immune cells including LAP positive T-cells and LAP positive platelets, with high efficiency.

Solution to Problem

The cell adsorption material of the present invention that solves the above-described problems is composed of the following configurations.

A cell adsorption material comprises fibers or particles of a water-insoluble carrier to which at least one nitrogen-containing compound is bound. The nitrogen-containing compound is selected from the group consisting of polyamines and aliphatic amines. The fibers or the particles have a region A and a region B, the region A being a region within 1.0 $\mu$m from an outermost surface of a cross-sectional surface of the fibers or the particles, the region B being a region within 0.5 $\mu$m radius from a center of gravity of the cross-sectional surface of the fibers or the particles, a spectral intensity of $^{26}CN^-$ satisfies formulae (1) and (2) below when a spectral intensity of $^{25}C_2H^-$ in each of the regions measured by Time of Flight Secondary Ion Mass Spectrometry is set to be 1:

$$0.5 \leq \text{the spectral intensity of } ^{26}CN^- \text{ in the region A} \leq 3.0 \ ... \ \text{Formula (1)};$$

3.0 $\leq$ the spectral intensity of $^{26}CN^-$ in the region A/the spectral intensity of $^{26}CN^-$ in the region B $\leq$ 20.0    Formula (2).

Advantageous Effects of Invention

The cell adsorption material of the present invention allows adsorbing and removing cells, such as LAP positive immune cells including LAP positive T-cells and LAP positive platelets, with high efficiency.

Brief Description of Drawings

Fig. 1 is a vertical cross-sectional view of an exemplary radial flow type cell adsorption column.
Fig. 2 is an exemplary cross-sectional surface of a cell adsorption material in Example 1 in which a mass spectrum of $^{26}CN^-$ is mapped and a spectral intensity of $^{26}CN^-$ is analyzed.
Fig. 3 is an enlarged view of Fig. 2.
Fig. 4 is an exemplary plot profile of the spectral intensity of $^{26}CN^-$ on a cross-sectional surface of the cell adsorption material.
Fig. 5 is an exemplary analysis of solidity on the cross-sectional surface of the cell adsorption material.

Description of Embodiments

The inventors have found that cells, such as LAP positive immune cells, can be adsorbed and removed by binding a polyamine or an aliphatic amine to a fibrous or particulate water-insoluble carrier and causing a spectral intensity of $^{26}CN^-$ to fall within a predetermined range when a spectral intensity of $^{21}C_2H^-$ on a cross-sectional surface of the above-described fibers or the above-described particles measured by Time of Flight Secondary Ion Mass Spectrometry is set to be 1.

That is, the present invention is a cell adsorption material that comprises fibers or particles of a water-insoluble carrier to which at least one nitrogen-containing compound selected from the group consisting of polyamines and aliphatic amines is bound, and in which the above-described fibers or the above-described particles have a region A that is a region within 1.0 μm from the outermost surface of a cross-sectional surface of the fibers or particles and a region B that is a region within 0.5 μm radius from a center of gravity of the cross-sectional surface of the fibers or particles, and a spectral intensity of $^{26}CN^-$ satisfies the following formulae (1) and (2) when a spectral intensity of $^{25}C_2H^-$ in each of the regions described above measured by Time of Flight Secondary Ion Mass Spectrometry is set to be 1.

$$0.5 \leq \text{spectral intensity of } ^{26}CN^- \text{ in region A described above} \leq 3.0 \ldots$$

Formula (1)

3.0 ≤ spectral intensity of $^{26}CN^-$ in region A described above/spectral intensity of $^{26}CN^-$ in region B described above ≤ 20.0      Formula (2)

The present invention relates to a cell adsorption material for LAP positive immune cells and the like. The LAP positive immune cell means a cell to which LAP-TGF-β complexes are bound to a cell membrane surface. While the LAP positive immune cell specifically means a LAP positive T-cell and a LAP positive platelet, it is not limited to those described above as long as the LAP-TGF-β complexes are bound to the cell membrane surface.

The cell adsorption material of the present invention comprises fibers or particles of a water-insoluble carrier to which at least one nitrogen-containing compound selected from the group consisting of polyamines and aliphatic amines is bound.

For the nitrogen-containing compound, one kind may be used alone or a plurality of kinds may be used in combination. The nitrogen-containing compound may directly be bound to the water-insoluble carrier or may indirectly be bound to the water-insoluble carrier via a linker.

The nitrogen-containing compound in the cell adsorption material of the present invention is preferably a polyamine represented by General Formula (I) as it has a high affinity with LAP positive immune cells.

$$R^1R^2N\text{-}X\text{-}NR^3R^4 \ldots \qquad (I)$$

In General Formula (I), X is a saturated or unsaturated aliphatic hydrocarbon group having 2 to 20 carbon atoms, or a heteroatom-containing carbon chain in which 1 to 5 carbon atoms of a saturated or unsaturated aliphatic hydrocarbon group having 3 to 20 carbon atoms are replaced with a nitrogen atom, a hydrogen atom that bonds to the nitrogen atom may be replaced with an alkyl group or an alkyl group having an amino group. $R^1$ to $R^4$ are each independently a hydrogen atom or an alkyl group.

In General Formula (I), X is, for example, a saturated or unsaturated aliphatic hydrocarbon group having 2 to 20 (for example, 16 or less, 14 or less, 12 or less, 10 or less, 8 or less, 6 or less, and 4 or less) carbon atoms. In General Formula (I), X is, for example, a heteroatom-containing carbon chain in which 1 to 5 (for example, 1 to 3) carbon atoms of a saturated or unsaturated aliphatic hydrocarbon group having 3 to 20 (for example, 16 or less, 14 or less, 12 or less, and 10 or less) carbon atoms are replaced with a nitrogen atom. The hydrogen atom that bonds to the nitrogen atom may be replaced with an alkyl group or an alkyl group having an amino group (for example, an alkyl group having 1 to 6 (preferably, 1 to 4) carbon atoms). $R^1$ to $R^4$ are each independently a hydrogen atom or an alkyl group. The alkyl group, for example, has 1 to 6 (preferably, 1 to 4) carbon atoms. The aliphatic hydrocarbon group may be linear or may be branched.

The polyamine represented by General Formula (I) is more preferably a polyamine represented by any one of the following General Formulae (II) to (VII), as it has high reactivity with the water-insoluble carrier.

$$H_2N\text{-}(CH_2)_{p1}\text{-}NH_2 \ldots \qquad (II)$$

In General Formula (II), p1 is an integer from 2 to 12 (preferably, 2 to 6, 2 to 5, or 2 to 4), and the hydrogen atoms of the primary amino groups at both ends may be replaced with an alkyl group.

$$H_2N\text{-}(CH_2)_{p1}\text{-}NH\text{-}(CH_2)_{p2}\text{-}NH_2 \ldots \qquad (III)$$

In General Formula (III), p1 and p2 are each independently an integer from 2 to 5 (preferably, 2 to 4, 2 to 3, or 2), the hydrogen atom of the secondary amino group may be replaced with an alkyl group or an alkyl group having an amino group, and the hydrogen atoms of the primary amino groups at both ends may be replaced with an alkyl group.

$$H_2N\text{-}(CH_2)_{p1}\text{-}NH\text{-}(CH_2)_{p2}\text{-}NH\text{-}(CH_2)_{p3}\text{-}NH_2 ... \qquad (IV)$$

In General Formula (IV), p1, p2, and p3 are each independently an integer from 2 to 5 (preferably, 2 to 4, 2 to 3, or 2), the hydrogen atoms of the secondary amino groups may be each independently replaced with an alkyl group or an alkyl group having an amino group, and the hydrogen atoms of the primary amino groups at both ends may be replaced with an alkyl group.

$$H_2N\text{-}(CH_2)_{p1}\text{-}NH\text{-}(CH_2)_{p2}\text{-}NH\text{-}(CH_2)_{p3}\text{-}NH\text{-}(CH_2)_{p4}\text{-}NH_2 ... \qquad (V)$$

In General Formula (V), p1, p2, p3, and p4 are each independently an integer from 2 to 5 (preferably, 2 to 4, 2 to 3, or 2), the sum of p1, p2, p3, and p4 is 17 or less, the hydrogen atoms of the secondary amino groups may be each independently replaced with an alkyl group or an alkyl group having an amino group, and the hydrogen atoms of the primary amino groups at both ends may be replaced with an alkyl group.

$$H_2N\text{-}(CH_2)_{p1}\text{-}NH\text{-}(CH_2)_{p2}\text{-}NH\text{-}(CH_2)_{p3}\text{-}NH\text{-}(CH_2)_{p4}\text{-}NH\text{-}(CH_2)_{p5}\text{-}NH_2 ... \qquad (VI)$$

In General Formula (VI), p1, p2, p3, p4, and p5 are each independently an integer from 2 to 5 (preferably, 2 to 4, 2 to 3, or 2), the sum of p1, p2, p3, p4, and p5 is 16 or less, the hydrogen atoms of the secondary amino groups may be each independently replaced with an alkyl group or an alkyl group having an amino group, and the hydrogen atoms of the primary amino groups at both ends may be replaced with an alkyl group.

$$H_2N\text{-}(CH_2)_{p1}\text{-}NH\text{-}(CH_2)_{p2}\text{-}NH\text{-}(CH_2)_{p3}\text{-}NH\text{-}(CH_2)_{p4}\text{-}NH\text{-}(CH_2)_{p5}\text{-}NH\text{-}(CH_2)_{p6}\text{-}NH_2 ... \qquad (VII)$$

In General Formula (VII), p1, p2, p3, p4, p5, and p6 are each independently an integer from 2 to 5 (preferably, 2 to 4, 2 to 3, or 2), the sum of p1, p2, p3, p4, p5, and p6 is 15 or less, the hydrogen atoms of the secondary amino groups may be each independently replaced with an alkyl group or an alkyl group having an amino group, and the hydrogen atoms of the primary amino groups at both ends may be replaced with an alkyl group.

In General Formulae (II) to (VII), the number of carbon atoms of "an alkyl group or an alkyl group having an amino group" that can bond to the nitrogen atom of the secondary amino group is, for example, 1 to 6, preferably 1 to 5, more preferably 1 to 4, and further more preferably 1 to 3. In General Formulae (II) to (VII), the number of carbon atoms of "an alkyl group" that can bond to the nitrogen atom of the primary amino groups at both ends is, for example, 1 to 6, preferably 1 to 5, preferably 1 to 4, and preferably 1 to 3. These "alkyl groups" are preferred to be linear or branched chain.

Examples of the polyamine represented by General Formula (I) include, for example, ethylenediamine, N-ethylethylenediamine, diethylenetriamine (hereinafter referred to as "DETA"), N-ethyldiethylenetriamine, triethylenetetramine, and tetraethylenepentamine. Besides them, the polyamines include, for example, 3,3'-diaminodipropylamine, 1,3-diaminopropane, norspermidine, homospermidine, aminopropylcadaverine, amino butyl cadaverine, norspermine, thermospermine, aminopropylhomospermidine, canavalmine, homospermine, aminopentylnorspermidine, N,N-bis(aminopropyl)cadaverine, caldopentamine, homocaldopentamine, thermopentamine, caldohexamine, homocaldohexamine, thermohexamine, homothermohexamine, $N^4$-aminopropylnorspermidine, $N^4$-aminopropylspermidine, and $N^4$-aminopropylnorspermine.

In the cell adsorption material of the present invention, when the nitrogen-containing compound is an aliphatic amine, it is preferred to be a primary aliphatic amine represented by General Formula (VIII) or a secondary aliphatic amine represented by General Formula (IX) from the standpoint of reactivity with the water-insoluble carrier and affinity with the LAP positive immune cells.

$$NH_2R^5 ... \qquad (VIII)$$

In General Formula (VIII), $R^5$ is a saturated or unsaturated aliphatic hydrocarbon group having 1 to 12 carbon atoms.

$$NHR^6R^7 ... \qquad (IX)$$

In General Formula (IX), $R^6$ and $R^7$ are each independently a saturated or unsaturated aliphatic hydrocarbon group having 1 to 12 carbon atoms.

In the aliphatic amine represented by General Formula (VIII) or (IX), the aliphatic hydrocarbon group is preferred to be a linear or branched chain aliphatic hydrocarbon group, and is preferred to be a linear or branched chain saturated aliphatic hydrocarbon group. The number of carbon atoms of the aliphatic hydrocarbon group is preferably 1 to 8, preferably 1 to 6, and preferably 1 to 4.

Examples of the aliphatic amine preferred as the nitrogen-containing compound include, for example, monoalkylamines, such as ethylamine, propylamine, butylamine, pentylamine, hexylamine, heptylamine, octylamine, nonylamine, and decylamine, and dialkylamines, such as diethylamine, dipropylamine, dibutylamine, diheptylamine, dioctylamine, and dicyclohexylamine.

Examples of other preferred nitrogen-containing compounds include, for example, ethylamine, ethylenediamine, diethylamine, N-ethylethylenediamine, DETA, N-ethyldiethylenetriamine, triethylenetetramine, or tetraethylenepentamine. Among these, ethylenediamine, DETA, triethylenetetramine, or tetraethylenepentamine is preferred as the linear nitrogen-containing compound with primary amines at both ends has high reactivity. For the nitrogen-containing compound, those commercially available or those manufactured by a known method or an equivalent method thereof are usable.

When the polyamine represented by General Formula (I) is used as the nitrogen-containing compound, a plurality of amino groups may bind to the water-insoluble carrier to form a cross-linkage structure. That is, when the polyamine represented by General Formula (I) is bound to the water-insoluble carrier as the nitrogen-containing compound, at least two amino groups in the polyamine binding to the water-insoluble carrier forms the cross-linkage structure.

Methods for binding the nitrogen-containing compound to the water-insoluble carrier include, for example, a method that covalently binds the nitrogen-containing compound to a surface of the water-insoluble carrier via a linker by a chemical method.

For the covalent bind when the nitrogen-containing compound and the water-insoluble carrier are directly in covalent bind, it is preferred to be one that has an electrically neutral chemical bond, such as an amide bond, a urea bond, an ether bond, or an ester bond, and more preferred to be one that has an amide bond or a urea bond, since they have high reactivity with the nitrogen-containing compound.

When the nitrogen-containing compound is bound to the water-insoluble carrier via a linker, that is, when the nitrogen-containing compound is bound to the linker by the above-described covalent bind, the linker before the nitrogen-containing compound is bound to the water-insoluble carrier via the linker, that is, the linker before reaction is preferred to have a reactive functional group from the standpoint of improving introducing efficiency of the nitrogen-containing compound to the water-insoluble carrier. Examples of the reactive functional group that the linker before the reaction has include, for example, an active halogen group, such as a halomethyl group, a haloacetyl group, a haloacetamidomethyl group, or a halogenated alkyl group, an epoxy group, a carboxyl group, an isocyanate group, a thioisocyanate group, or an acid anhydride group. Among these, the active halogen group (in particular, the haloacetyl group) is preferred because it is easily manufactured, has appropriately high reactivity, can perform an immobilizing reaction of the nitrogen-containing compound in a mild condition, and generates a chemically stabilized covalent bind. As examples of the water-insoluble carrier to which the linker having the reactive functional group is introduced include, for example, a polystyrene to which a chloroacetamidomethyl group is added, a polysulfone to which a chloroacetamidomethyl group is added, or a polyetherimide to which a chloroacetamidomethyl group is added. It should be noted that these polymers are soluble in an organic solvent, thereby having an advantage of easy molding. While a rough indication of the introduction amount of the linker depends on a structure of the linker, for example, in the case with a chloroacetamidomethyl group, it is preferred to be 1 mmol or more and 10 mmol or less per 1 g of water-insoluble carrier. Since the introduction amount of the linker affects a binding amount of the nitrogen-containing compound to the water-insoluble carrier, it is more preferred to be 2 mmol or more, and further preferred to be 3 mmol or more. From the standpoint of maintaining the strength of the water-insoluble carrier, the introduction amount of the linker is more preferably 8 mmol or less, and further preferably 7 mmol or less. Any preferable lower-limit value may be combined with any preferable upper-limit value.

Examples of the methods to introduce the linker having the reactive functional group to the water-insoluble carrier include a method that reacts the linker having the reactive functional group with the water-insoluble carrier in advance. For example, when the water-insoluble carrier is a polystyrene and the linker having the reactive functional group is a chloroacetamidomethyl group, reacting the polystyrene with N-methylol-α-chloroacetamide (hereinafter referred to as "NMCA") allows obtaining the polystyrene to which the chloroacetamidomethyl group is added.

The nitrogen-containing compound in the cell adsorption material of the present invention is preferred to be bound to the water-insoluble carrier via an amino group (or a nitrogen atom) of the nitrogen-containing compound.

When the nitrogen-containing compound binds to the water-insoluble carrier, primary amino groups, secondary amino groups, tertiary amino groups, and/or quaternary amino groups exist in the nitrogen-containing compound after binding depending on binding positions in the nitrogen-containing compound. For example, when the polyamine represented by General Formula (I) binds to the water-insoluble carrier, primary amino groups, secondary amino groups, tertiary amino groups, and/or quaternary amino groups exist in the polyamine after binding depending on the binding positions in the polyamine.

The amino group in the cell adsorption material includes thus occurred primary amino groups, secondary amino group, tertiary amino groups, and quaternary amino groups derived from the nitrogen-containing compound. When the nitrogen-containing compound is bound to the water-insoluble carrier via the linker, the amino group in the cell adsorption material includes the primary amino groups, the secondary amino groups, the tertiary amino groups, and the quaternary amino groups derived from the linker. In this description, an "amino group amount" means the amount of the primary amino groups, the secondary amino groups, the tertiary amino groups, and the quaternary amino groups in the cell adsorption material.

The amino group amount in the cell adsorption material of the present invention is preferably 20 $\mu$mol/g or more and 200 $\mu$mol/g or less. From the standpoint of imparting sufficient cell adsorption performance, the amino group amount is more preferred to be 30 $\mu$mol/g or more, and further preferred to be 50 $\mu$mol/g or more. From the standpoint of suppressing adsorption of monocytes and granulocytes, which are a kind of leukocytes, and from the standpoint of being able to suppress adsorption of anticoagulant agents, such as heparin, the amino group amount is more preferred to be 150 $\mu$mol/g or less, and further preferred to be 100 $\mu$mol/g or less. Any preferable lower-limit value may be combined with any preferable upper-limit value.

The amino group amount in the cell adsorption material can be obtained as the sum of an amount of the primary amino groups, an amount of the secondary amino groups, an amount of the tertiary amino groups, and an amount of the quaternary amino groups (quaternary ammonium groups) by measuring the amino groups using, for example, acid-base back titration. That is, by using an excessive amount of a sodium hydroxide aqueous solution, the amino groups to which salts in the cell adsorption material are added are desalinated, and the cell adsorption material is sufficiently cleaned to be neutral and dried. The obtained amino groups in the cell adsorption material is reacted with a constant amount of a standard solution containing an excessive acid. At this time, titrating an amount of the acid that remains without reacting with the amino groups with the standard solution containing bases allows obtaining the amino group amount. Further specifically, the amino group amount can be obtained by a method described in "Measurement of Amino Group Amount" described later. Note that, while this method can measure an amount including the quaternary amino groups, the quaternary amino group is not structurally contained in the used nitrogen-containing compound in the examples, and therefore, it is the sum of the primary amino group amount, the secondary amino group amount, and the tertiary amino group amount.

The amino group amount in the cell adsorption material can be controlled by, for example, adjusting the binding amount of the reactive functional groups, the kind of the nitrogen-containing compound, and the use amount of the nitrogen-containing compound, introduced to the water-insoluble carrier directly or via the linker. The binding amount of the reactive functional groups introduced to the water-insoluble carrier via the linker can be controlled by, for example, the kind of the reactive functional group that the linker has, the kind of the solvent, or reactive conditions, such as an immersion temperature or an immersion period. For example, when the water-insoluble carrier contains polyaromatic vinyl, it is also possible to control bindable positions between the water-insoluble carrier and the reactive functional group or the linker having the reactive functional group by using crosslinking agents.

When the nitrogen-containing compound is bound via a linker having an amide group in the cell adsorption material of the present invention, a ratio of the amino group amount to an amide group amount (amino group/amide group ratio) in the cell adsorption material is preferably 0.008 or more and 0.080 or less. From the standpoint of imparting a sufficient cell adsorption performance, the ratio of the amino group amount to the amide group amount is more preferably 0.010 or more, further preferably 0.015 or more, and particularly preferably 0.020 or more. From the standpoint of suppressing the adsorption of monocytes and granulocytes, which are a kind of leukocytes, thereby allowing removal of the cells, such as LAP positive immune cells with high efficiency, and suppressing the adsorption of anticoagulant agents, such as heparin, the ratio of the amino group amount to the amide group amount is more preferably 0.070 or less, further preferably 0.060 or less, and particularly preferably 0.050 or less. Any preferable lower limit may be combined with any preferable upper-limit value.

The amide group amount in the cell adsorption material can be measured by, for example, titrating after hydrolyzing the amide groups in the cell adsorption material. Specifically, it can be obtained by a method described in "Measurement of Ratio of Amino Group/Amide Group" described later.

The amide group amount in the cell adsorption material can be controlled by, for example, using linkers having an amide group as a reactive functional group and adjusting an introduction amount of the linkers to the water-insoluble carrier. As a more specific example, by introducing NMCA as a linker having a reactive functional group and having an amide group to the water-insoluble carrier, the amide group can be added to the water-insoluble carrier.

From the standpoint of increasing the introduction amount of the nitrogen-containing compound to improve the cell adsorption performance in the cell adsorption material of the present invention, the amide group amount is preferably 2,500 $\mu$mol/g or more, and is more preferably 2,800 $\mu$mol/g or more. Meanwhile, from the standpoint of suppressing increase of surface roughness of the water-insoluble carrier by linker introduction reaction to suppress increase of non-specific adsorption, the amide group amount is preferably 5,000 $\mu$mol/g or less, and more preferably 4,000 $\mu$mol/g or

less. Any preferable lower limit may be combined with any preferable upper-limit value.

"The water-insoluble carrier" means a carrier that does not change its mass when it is immersed in water having a normal temperature (25°C), specifically, it is preferred to be a carrier having a mass change of 5% or less when it is immersed in the water of 25°C for one hour.

Examples of the water-insoluble carrier materials preferably include, for example, polyaromatic vinyl compounds typified by polystyrenes, polyethersulfones, polysulfones, polyarylethersulfones, polyetherimides, polyimides, polyamides, or polyphenylene sulfides. For the material of the water-insoluble carrier, one commercially available or one manufactured by a known method or an equivalent method thereof can be used. These polymer materials are materials that do not substantially have a hydroxyl group that is said to easily activate complements when contacting blood. Among these, polystyrenes are preferred as the material of the water-insoluble carrier because it has many aromatic rings per unit mass and the nitrogen-containing compound is easily immobilized to polystyrenes. These polymer materials may be used alone or may be used in combination of a plurality of kinds. The water-insoluble carrier is preferred to be a polymer material comprising a polyaromatic vinyl compound, for example, a polystyrene. The water-insoluble carrier is preferred to be a copolymer of a polystyrene and a polyolefin, for example, a copolymer of a polystyrene and a polyethylene or a copolymer of a polystyrene and a polypropylene in that it is easy to introduce linkers, such as an active halogen group, for immobilizing a nitrogen-containing compound to a polystyrene part, it is easy to handle due to strength reinforcement by a polyolefin part, and in terms of chemical resistance. The polymer material may be one that is blended or alloyed, and in particular, a polymer alloy of a polystyrene and a polyolefin, for example, a polymer alloy of a polystyrene and a polyethylene or a polymer alloy of a polystyrene and a polypropylene is preferred from the standpoint that it has chemical resistance and easily retains its physical shape. Among these, the polymer alloy of a polystyrene and a polypropylene that has a proven use in a blood extracorporeal circulation therapy is preferred. It is preferred that the water-insoluble carrier to be used does not substantially have an amino group.

An immobilizing amount of nitrogen-containing compound in the cell adsorption material can be controlled by, for example, adjusting an introduction amount of the reactive functional groups or the linkers having the reactive functional groups to the water-insoluble carrier, a kind of the nitrogen-containing compound, or use amount of the nitrogen-containing compound. The introduction amount of the linkers having the reactive functional groups can be controlled by, for example, a kind of the linkers having the reactive functional groups, a kind of the solvent, or reactive conditions, such as an immersion temperature or an immersion period. For example, when the water-insoluble carrier contains polyaromatic vinyl, it is also possible to control bindable positions of the reactive functional group or the linker having the reactive functional group by using crosslinking agents.

The water-insoluble carrier in the cell adsorption material of the present invention is a fiber or a particle. In particular, the fiber is preferred in that the fiber can have an increased area in contact with blood while securing a blood flow passage by high-order processing.

When the water-insoluble carrier is a fiber, a sea-island type composite fiber is preferred, and from a standpoint of retaining strength as a material, a sea-island type composite fiber whose island is a reinforcing material and sea is an alloy of a water-insoluble polymer and a reinforcing material is preferred, and furthermore, a sea-island type composite fiber in which island is a polypropylene and sea is an alloy of a polystyrene and a polypropylene is preferred. Examples of the reinforcing materials include, for example, polyamides, polyacrylonitriles, polyethylenes, polypropylenes, nylons, polymethyl methacrylates, or polytetrafluoroethylenes. Among these, polypropylenes are preferred as they are high in chemical resistance and also high in thermoplasticity. These polymers may be used alone or may be used in combination of a plurality of kinds.

When the water-insoluble carrier is the fiber, it is preferred to be a knitted fabric as its high-order processed product. The knitted fabric can secure a blood flow passage by controlling its stitch, thereby allowing reduced pressure loss when blood passes through the fibers. When the knitted fabric is formed by doubling the fibers, the number of doublings is preferably 10 or more and 100 or less, and more preferably 30 or more and to 80 or less. When the number of doublings is 100 or less, the LAP positive immune cells easily flows into a fiber bundle, thereby improving the adsorption performance. When the number of doublings is 10 or more, maintainability of the form of the knitted fabric improves. Note that, any preferable lower-limit value may be combined with any preferable upper-limit value. Note that when the knitted fabric is manufactured or when the fibers are combined, the fibers to which the nitrogen-containing compound is bound, that is the cell adsorption material, and the fibers to which the nitrogen-containing compound is not bound may be combined.

A major axis of the fibers or the particles of the water-insoluble carrier in the cell adsorption material of the present invention is preferably 15 $\mu$m or more and 50 $\mu$m or less.

When the major axis of the fibers or the particles as the water-insoluble carrier in the cell adsorption material is 15 $\mu$m or more and 50 $\mu$m or less, it is presumed that non-specific adsorption of, for example, platelets and leukocytes is suppressed and adsorption performance of LAP positive immune cells is improved because the area of the cell adsorption material that is in contact with blood per unit volume can be increased while maintaining filling density of the cell adsorption material in the column at an appropriate magnitude. Therefore, the major axis of the fibers or the particles as the water-insoluble carrier in the cell adsorption material is more preferably 17 $\mu$m or more, further preferably 18 $\mu$m or more, and particularly preferably 19 $\mu$m or more. Meanwhile, the major axis of the fibers or the particles is more preferably 40 $\mu$m or less, further preferably 35 $\mu$m or less, and particularly preferably 30 $\mu$m or less. Any preferable lower-limit value may be combined with any preferable upper-limit value.

"The major axis of the fibers" can be measured by taking a photograph of a cross-sectional surface perpendicular to an elongation direction of the fiber at a magnification of 1,000-fold or more and 3,000-fold or less using a scanning electron microscope or the like and analyzing the obtained image of the fiber cross-sectional surface. Specifically, the measurement can be performed by a method described in "Measurement of Major Axis of Fibers" described later.

For "the major axis of the particles," ten sample groups of the particles are randomly extracted and one photograph for each sample group is taken at a magnitude of 1,000-fold or more and 3,000-fold or less using a scanning electron microscope or the like. Next, major axes of the ten particles per photograph are measured in a similar way to the major axis measurement of the fibers cross-sectional surface. Calculating the average values of those values (the average value of major axes of 100 particles in total) obtains "the major axis of the particles." Note that, for the average value, a value rounded to the nearest whole number is used.

The cell adsorption material of the present invention has a region A that is a region within 1.0 $\mu$m from the outermost surface of the cross-sectional surface of the fibers or the particles as the cell adsorption material and a region B that is a region within 0.5 $\mu$m radius from a center of gravity of the cross-sectional surface of the fibers or the particles as the cell adsorption material, and the spectral intensity of $^{26}CN^-$ satisfies the following formulae (1) and (2) when the spectral intensity of $^{25}C_2H^-$ in each region measured by Time of Flight Secondary Ion Mass Spectrometry is set to be 1.

$$0.5 \leq \text{spectral intensity of } ^{26}CN^- \text{ in region A} \leq 3.0 \ ... \ \text{Formula (1)}$$

$$3.0 \leq \text{spectral intensity of } ^{26}CN^- \text{ in region A/spectral intensity of } ^{26}CN^- \text{ in region B} \leq 20.0 \qquad \text{Formula (2)}$$

When the spectral intensity of $^{26}CN^-$ in the region A is less than 0.5, it is presumed that the amount of the nitrogen-containing compound bound to the surface of the water-insoluble carrier is small, and therefore, the adsorption performance of cells, such as LAP positive immune cells, is not sufficiently obtained. Meanwhile, when the spectral intensity of $^{26}CN^-$ in the region A exceeds 3.0, it is presumed that the amount of the nitrogen-containing compound bound to the surface of the water-insoluble carrier is excessive, and therefore, adsorption of cells, such as LAP positive immune cells, may be inhibited by adsorption of monocytes, granulocytes, and the like, which are a kind of leukocytes. Adsorption of anticoagulant agents, such as heparin, increases, thereby raising a concern that blood coagulation is more likely to occur. When the spectral intensity of $^{26}CN^-$ in the region A/the spectral intensity of $^{26}CN^-$ in the region B is less than 3.0, adsorption performance of cells, such as LAP positive immune cells, is not sufficiently obtained. Meanwhile when the spectral intensity of $^{26}CN^-$ in the region A/the spectral intensity of $^{26}CN^-$ in the region B exceeds 20.0, it is presumed that the amount of the nitrogen-containing compound bound to the surface of the water-insoluble carrier is excessive, and therefore, adsorption of cells, such as LAP positive immune cells, may be inhibited by adsorption of monocytes, granulocytes, and the like, which are a kind of leukocytes, similarly to the case where the spectral intensity of $^{26}CN^-$ in the region A exceeds 2.0. Accordingly, it is presumed that the spectral intensity of $^{26}CN^-$ on the cross-sectional surface of the fibers or the particles as the cell adsorption material is required to fulfill Formula (1) and Formula (2). The spectral intensity of $^{26}CN^-$ in the region A is preferably 0.6 or more, more preferably 0.9 or more, and further preferably 1.2 or more. At the same time, it is preferably 2.5 or less, more preferably 2.0 or less, further preferably 1.8 or less, and particularly preferably 1.6 or less. The spectral intensity of $^{26}CN^-$ in the region A/the spectral intensity of $^{26}CN^-$ in the region B is preferably 5.0 or more, and more preferably 6.0 or more. At the same time, it is preferably 15.0 or less, more preferably 12.0 or less, further preferably 9.0 or less, and particularly preferably 7.0 or less. Any preferable lower-limit value may be combined with any preferable upper-limit value.

"The spectral intensities of $^{26}CN^-$ in the region A and the region B" can be obtained by measuring a mass spectrum of $^{25}C_2H^-$ and a mass spectrum of $^{26}CN^-$ on the cross-sectional surface of the cell adsorption material by Time of Flight Secondary Ion Mass Spectrometry (TOF-SIMS method), mapping each of the intensities, and analyzing them. More specifically, they can be obtained by a method described in "Measurement of Spectral Intensity of $^{26}CN^-$" described later.

The spectral intensity of $^{26}CN^-$ in the region A and the spectral intensity of $^{26}CN^-$ in the region B can be controlled by the introduction amount of the nitrogen-containing compound to the water-insoluble carrier, the kind of the reaction solvent when the nitrogen-containing compound is bound to the water-insoluble carrier, and the like. For example, the lower the affinity between the reaction solvent and the water-insoluble carrier is, the smaller the spectral intensity of $^{26}CN^-$ in the region B becomes.

The fibers or the particles as the cell adsorption material of the present invention is preferred to have a modal pore radius of 10 nm or more and 100 nm or less, and a total volume of pores with a pore radius of 10 nm or more and 250 nm or less with respect to a total volume of all the pores in the cell adsorption material is preferred to be 50% or more and 100% or less. "The modal pore radius" in this description means the most frequent pore radius when frequency of the pore radius of the fibers or the particles as the cell adsorption material is measured. Note that the total volume of all the pores of the cell adsorption material means the sum of volumes of all the pores observed during the measurement. The total volume of the pores with a pore radius of 10 nm or more and 250 nm or less means the sum of volumes of all the pores having the corresponding radius. The total volume of the pores with a pore radius of 10 nm or more and 250 nm or less with respect to the total volume of all the pores in the cell adsorption material, that is, "the total volume of the pores with a pore radius of 10 nm or more and 250 nm or less"/"the total volume of all the pores in the cell adsorption material" is referred to as a total volume ratio.

The reason and mechanism that the advantageous effects of the invention can be obtained when the modal pore radius and the total volume ratio of the cell adsorption material fall within the predetermined ranges are presumed as follows. When the modal pore radius of the cell adsorption material is less than 10 nm, proteins are easily adsorbed, and thus, non-specific adsorption of immune cells may be accelerated. Meanwhile, when the modal pore radius of the cell adsorption material exceeds 100 nm, unevenness on the surface of the cell adsorption material is reduced, and thus, adsorption performance of cells, such as LAP positive immune cells, may be reduced. When the proportion occupied by the total volume of the pores with a pore radius of 10 nm or more and 250 nm or less with respect to the total volume of all the pores in the cell adsorption material is less than 50%, non-specific adsorption of proteins and immune cells may be similarly induced. Accordingly, it is presumed that the modal pore radius and the total volume ratio of the cell adsorption material are preferred to fall within the predetermined ranges.

The modal pore radius and the total volume ratio can be controlled by immersing the water-insoluble carrier in an organic solvent, the kind of the reaction solvent when the nitrogen-containing compound is bound to the water-insoluble carrier, and the like. For example, the higher the affinity between the reaction solvent and the water-insoluble carrier is, the smaller the modal pore radius and the total volume ratio become.

The modal pore radius of the fibers or the particles as the cell adsorption material is more preferably 15 nm or more, further preferably 20 nm or more, further more preferably 30 nm or more, and particularly preferably 40 nm or more. At the same time, the modal pore radius is more preferably 95 nm or less, further preferably 85 nm or less, and particularly preferably 75 nm or less. Any preferable lower-limit value may be combined with any preferable upper-limit value. The proportion occupied by the total volume of the pores with a pore radius of 10 nm or more and 250 nm or less with respect to the total volume of all the pores in the cell adsorption material, that is, the total volume ratio is more preferably 70% or more, further preferably 80% or more, and particularly preferably 90% or more.

"The modal pore radius," "the total volume of the pores with a pore radius of 10 nm or more and 250 nm or less," and "the total volume of all the pores in the cell adsorption material" can be calculated by measuring Differential Scanning Calorimetry (DSC method) and analyzing it. Specifically, they can be obtained by a method described in "Measurement of Modal Pore Radius and Total Volume Ratio" described later.

The roundness of the cross-sectional surface of the fibers or the particles as the cell adsorption material of the present invention is preferred to have roundness of 0.75 or more and 0.95 or less. The roundness of the cross-sectional surface of the cell adsorption material is more preferably 0.77 or more, and further preferably 0.80 or more. At the same time, the roundness is more preferably 0.90 or less. Any preferable lower-limit value may be combined with any preferable upper-limit value. When the roundness of the cross-sectional surface is in the range of 0.75 or more and 0.95 or less, it is presumed that non-specific adsorption of monocytes and granulocytes to the cell adsorption material is suppressed and LAP positive immune cells can be removed with high efficiency.

"The roundness" is an indicator that represents how much the cell adsorption material as a target circular body deviates from the perfect circle. The roundness takes a value from 0.00 or more to 1.00 or less, and it means that the closer the value is to 1.00, the closer the shape of the target circular body is to the perfect circle. The roundness can be calculated by analyzing an image of the fiber cross-sectional surface or the whole particles with image analysis software. Specifically, it can be calculated by a method described in "Measurement of Roundness" described later.

The roundness can be controlled by immersing the water-insoluble carrier in an organic solvent, the kind of the reaction solvent when the nitrogen-containing compound is bound to the water-insoluble carrier, and the like. For example, the lower the affinity between the reaction solvent and the water-insoluble carrier is, the more the roundness becomes.

The solidity of the cross-sectional surface of the fibers or the particles as the cell adsorption material of the present invention is preferred to have solidity of 0.92 or more and 1.00 or less. Reducing the unevenness on the surface of the cell adsorption material suppresses non-specific adsorption of monocytes and granulocytes to the cell adsorption material and thus improves the cell adsorption performance, and therefore, the solidity of the cross-sectional surface of the cell adsorption material is more preferably 0.94 or more, and further preferably 0.96 or more.

"The solidity" is an indicator that represents scarcity of depressed portions on the cross-sectional surface of the cell adsorption material as the target form. The solidity takes a value from 0.00 or more to 1.00 or less, that is, the upper limit is 1.00, and it means the closer the value is to 1.00, the less the depressed portions of the target form has. The solidity can be calculated by analyzing an image of the cross-sectional surface of the fibers or the particles with image analysis software. Specifically, it can be calculated by a method described in "Measurement of Solidity" described later.

The solidity can be controlled by immersing the water-insoluble carrier in an organic solvent, the kind of the reaction solvent when the nitrogen-containing compound is bound to the water-insoluble carrier, and the like, similarly to the roundness. For example, the lower the affinity between the reaction solvent and the water-insoluble carrier is, the more the solidity becomes.

The surface of the fibers or the particles of the water-insoluble carrier as the adsorption material of the present invention is preferred to have arithmetic mean roughness of 0.01 μm or more and 1.0 μm or less.

"The arithmetic mean roughness" is a value obtained by the following Formula (3) when only a reference length L is extracted in a direction of its average line from a roughness curved line, and the x-axis is set in a direction of this average line of the extracted part, the y-axis is set in a direction of vertical magnification, and the roughness curved line is represented as y = f(x), which means the arithmetic mean roughness (Ra) in Japanese Industrial Standard B 0601-2001. The arithmetic mean roughness can be measured with, for example, a shape measurement laser microscope. For the measurement conditions, it is preferred to be performed in a condition where the cell adsorption material is dry. When there is an orientation as fibers have, a value in a longitudinal direction is measured.

[Math. 1]

$$ R a = \frac{1}{L} \int_0^L | f (x) | d x \quad \cdots \quad \text{Formula (3)} $$

The arithmetic mean roughness of the surface of the water-insoluble carrier is more preferably 0.01 μm or more and 1.00 μm or less. When the arithmetic mean roughness of the surface of the cell adsorption material is in the range of 0.01 μm or more and 1.00 μm or less, it is presumed that maintaining adsorption performance of LAP positive cells and suppressing non-specific adsorption of monocytes and granulocytes are possible. Therefore, the arithmetic mean roughness of the surface of the water-insoluble carrier is more preferably 0.05 μm or more, further preferably 0.10 μm or more, further more preferably 0.20 μm or more, particularly preferably 0.30 μm or more, and most preferably 0.40 μm or more. At the same time, the arithmetic mean roughness of the surface of the cell adsorption material is more preferably 0.90 μm or less, and further preferably 0.80 μm or less. Any preferable lower-limit value may be combined with any preferable upper-limit value. Note that the values of the preferable major axis of the fibers or the particles as the cell adsorption material, the preferable roundness and solidity of the cell adsorption material, and the preferable arithmetic mean roughness of the surface of the cell adsorption material may be conveniently combined.

The arithmetic mean roughness of the surface of the cell adsorption material can be controlled by immersing the water-insoluble carrier in an organic solvent and the like. For example, there is a method that immerses polymers obtained by mixing a polyaromatic vinyl compound and a polypropylene as the water-insoluble carrier in a solvent in which a part of the polyaromatic vinyl compound is soluble and the polypropylene is insoluble. The arithmetic mean roughness of the surface of the cell adsorption material can be controlled by the kind of the polymers, the molecular weight of the polymers, the kind of the solvent, the immersion temperature, the immersion period, and the like. Furthermore, for the polyaromatic vinyl, it is also possible to employ a method that, for example, controls the solubility in the solvent by introducing a crosslinking agent. Furthermore, the above-described reaction can be performed simultaneously with the introduction reaction of the nitrogen-containing compound.

The cell adsorption material of the present invention is preferably a cell adsorption material that adsorbs immune cells. It is preferably a cell adsorbent that adsorbs LAP positive immune cells as the immune cells. It is preferred that the cell adsorption material adsorbs LAP positive T-cells or LAP positive platelets among the LAP positive immune cells, and it is more preferred that the cell adsorption material adsorbs LAP positive CD4-positive T-cells or LAP positive CD42b-positive platelets.

In the cell adsorption material of the present invention, an adsorption rate of LAP positive T-cells is preferably 60% or more, more preferably 70% or more, and further preferably 80% or more. An adsorption rate of LAP positive platelets is preferably 70% or more, more preferably 80% or more, and further preferably 90% or more. Here, CD4-positive T-cells are subjects as the LAP positive T-cells, and CD42b-positive platelets are subjects as the platelets. Examples of a test system of the above-described adsorption rate include, for example, an adsorption column liquid passage type immune cell adsorption test (see Examples) using human blood. Examples of evaluation systems include, for example, an analysis (see Examples) by a flow cytometry using surface antigens of the immune cells as an index.

The cell adsorption column of the present invention has the cell adsorption material of the present invention built-in.

"The cell adsorption column" means one having at least a blood inlet portion, a housing portion, and a blood outlet portion, and the housing portion is filled with the cell adsorption material. Exemplary cell adsorption columns include, for example, a radial flow type cell adsorption column. As described above, the form of the cell adsorption material is preferably a fiber, and preferably a knitted fabric.

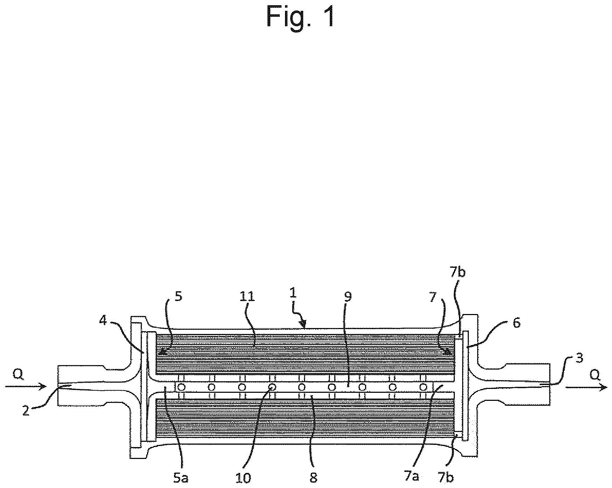

An exemplary configuration of an inside of the cell adsorption column of the present invention will be described along FIG. 1. In FIG. 1, reference numeral 1 denotes a container body, and there are an inflow port 2 and an outflow port 3 at a front end and a rear end in its longitudinal direction. The inflow port 2 has an inside where a filter 4 and a circular plate-shaped partition plate 5 are disposed. The outflow port 3 has an inside where a filter 6 and a circular plate-shaped partition plate 7 are disposed. Among the two partition plates 5 and 7, the partition plate 5 in the front side (inflow port side) has a center portion where an opening 5a is provided, and the partition plate 7 in the rear side has a center portion where a support protrusion 7a is provided. The partition plate 7 has an outer periphery where multiple through holes 7b are intermittently provided in the circumferential direction. Furthermore, one pipe 8 is bridged between the opening 5a of the partition plate 5 and the support protrusion 7a of the partition plate 7. The pipe 8 internally has a flow passage 9 that introduces blood and has a peripheral wall on which multiple through-holes 10 are provided. The pipe 8 has its front end communicating with the opening 5a of the partition plate 5 and has its rear end closed with the support protrusion 7a of the partition plate 7. The outer periphery of this pipe 8 is wound with a plurality of layers of a cell adsorption material 11 for many times. When this cell adsorption column is used for a circulation method, tubes are coupled to the inflow port 2 and the outflow port 3 that forms a circulation circuit with a blood pool. The blood taken out of the blood pool is supplied to the inflow port 2, target adsorption substances (LAP positive immune cells) are removed with the cell adsorption material 11 inside, and the blood is flown out of the outflow port 3 to circulate the blood so as to return to the blood pool again. In the column, the blood that entered the flow passage 9 through the filter 4 from the inflow port 2 moves through the flow passage 9 and sequentially infiltrates into the cell adsorption material 11 from the through-holes 10. The cell adsorption material 11, then, adsorbs cells and the like in the blood moving to any of the radial directions. The blood from which the cells and the like are removed flows out of the multiple through holes 7b on the outer periphery of the partition plate 7, and flows out of the outflow port 3 through the filter 6. While in the above-described example, the blood flows out of the through-holes 10 while flowing through the flow passage 9 inside the pipe 8 from the opening 5a, the moving direction of the blood in the cell adsorption column may be inverted from the above to supply the blood from the outflow port 3 and be flown out of the inflow port 2.

In order to increase the adsorption rate of LAP positive immune cells and the like, a blood linear velocity in the column is also important. That is, when the blood linear velocity is fast, it is possible that a sufficient interaction of LAP positive immune cells with the cell adsorption material becomes difficult. Meanwhile, when the blood linear velocity is slow, there may be a case where other blood components, such as platelets and proteins, non-specifically adhere to the cell adsorption material to inhibit the interaction between the cell adsorption material and LAP positive immune cells and the like. Accordingly, the maximum value of blood linear velocity in the cell adsorption material when a flow rate $S_{in}$ of the adsorption column inlet is 50 cm$^3$/minute is preferably 50 cm/minute or less, and preferably 25 cm/minute or less. The minimum value of blood linear velocity in the cell adsorption material when the flow rate of the cell adsorption column inlet is 50 cm$^3$/minute is preferably 0.1 cm/minute or more and preferably 0.5 cm/minute or more. Here, the blood linear velocity is obtained by a calculation, and, for example, in the case of the following radial flow type cell adsorption column, the maximum value ($V_{max}$) of the blood linear velocity in the cell adsorption material is calculated from a total area ($S_p$) of the openings that open on the side surface of the center pipe and the flow rate $S_{in}$ (50 cm$^3$/minute) of the cell adsorption column inlet by the following Formula (4).

$$V_{max}(cm/minute) = S_{in}(cm^3/minute)/S_p(cm^2) \ ... \ Formula \ (4)$$

The minimum value ($V_{min}$) is calculated from an area ($S_o$) of an outermost peripheral surface of the cell adsorption material wound around the center pipe and the flow rate $S_{in}$ (50 cm$^3$/minute) of the cell adsorption column inlet by the following Formula (5).

$$V_{min}(cm/minute) = S_{in}(cm^3/minute)/S_o(cm^2) \ ... \ Formula \ (5)$$

Furthermore, the cell adsorption column of the present invention is preferred to be a radial flow type cell adsorption column that includes a center pipe, a plate A, and a plate B. The center pipe has a side surface in the longitudinal direction where through-holes are provided for flowing out the supplied blood. The cell adsorption material is filled around the above-described center pipe. The plate A is communicated through the upstream end of the above-described center pipe for causing the above-described blood that flows in to pass inside the above-described center pipe and is disposed to prevent the above-described blood from contacting the cell adsorption material without passing through the above-described center pipe. The plate B is disposed to close the downstream end of the above-described center pipe and secure the cell adsorption material in a space around the above-described center pipe. This is to cause the blood to uniformly flow through the cell adsorption material. It should be noted that when an aperture ratio of the through-holes of the above-described center pipe is low, the pressure loss easily occurs in this part, and therefore, granulocytes, monocytes, and platelets are activated so that they are easily adhered non-specifically to the cell adsorption material. Therefore, the adsorption selectivity of LAP positive immune cells and the like may lower. When the aperture ratio is high, it is possible that problems, such as reduced strength of the pipe and easy occurrence of a short path at the through-holes near the blood inlet portion, arise. Accordingly, the aperture ratio of the through-holes is preferably 20% or more and 80% or less, and preferably 30% or more and 60% or less.

"The radial flow type" means the way blood flows inside the column. When blood is flown in the perpendicular direction to the inlet and the outlet of the column, and when there is a blood flow in the horizontal direction inside the column, it is referred to as a radial flow type.

"The aperture ratio of through-holes" means a value obtained by the following Formula (6).

Aperture ratio of through-holes (%) = sum of areas of through-holes formed on side surface in longitudinal direction of pipe/area of side surface of pipe $\times$ 100      Formula (6)

The cell adsorption column of the present invention can be used in a blood purification therapy. Using the cell adsorption column of the present invention as a column for blood purification allows suppressing non-specific adsorption of platelets, leukocytes, and the like and removing LAP positive immune cells and the like from blood with high efficiency. For example, extracorporeally circulating blood and passing the blood through the cell adsorption column of the present invention allows removing LAP positive immune cells and the like from blood with high efficiency. That is, the cell adsorption column of the present invention can be used as a column for extracorporeal circulation. More specifically, the cell adsorption column of the present invention can be used for a cancer therapy that removes LAP positive immune cells from blood of a cancer patient. It is also possible to be used in combination with a cell infusion treatment that activates dendritic cells, natural killer cells, and the like.

Examples

While the following describes the present invention with examples, the present invention is not limited to these examples.

<Measurement of Spectral Intensity of $^{26}CN^-$>

Fibers or particles of a water-insoluble carrier to which a nitrogen-containing compound is bound as a dried adsorption material were embedded in a resin, and a cross-sectional surface of the adsorption material was produced using a ultramicrotome. When the above-described water-insoluble carrier was a fiber, a cross-sectional surface perpendicular to an elongation direction of the fibers was produced, and when the above-described water-insoluble carrier was a particle, a cross-sectional surface perpendicular to a longitudinal direction of the particles was produced. A mass spectrum of $^{25}C_2H^-$ and a mass spectrum of $^{26}CN^-$ as a kind of a negative secondary ion discharged from the above-described cross-sectional surface by irradiation of primary ions were measured over the whole cross-sectional surface described above using TOF-SIMS method under the following conditions.

Measurement Equipment: TOF.SIMS 5 (manufactured by IONTOF GmbH)
Primary Ion: $Bi_3^{++}$
Primary Ion Accelerating Voltage: 30 kV
Pulse Width: 125.0 ns
Secondary Ion Polarity: positive and negative
Number of Scans: 96 scan/cycle
Cycle Time: 230 $\mu$s
Measuring Range: 50 $\times$ 50 $\mu m^2$
Mass Range (m/z): 0 to 800

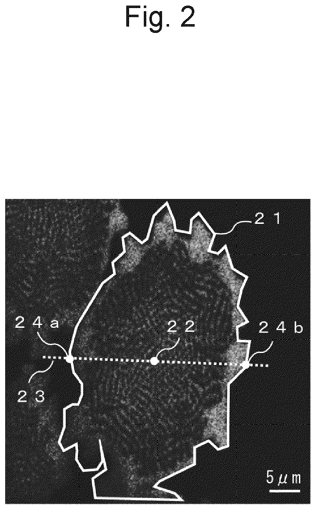

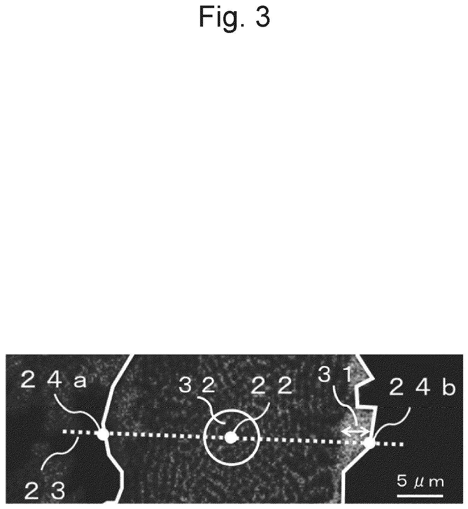

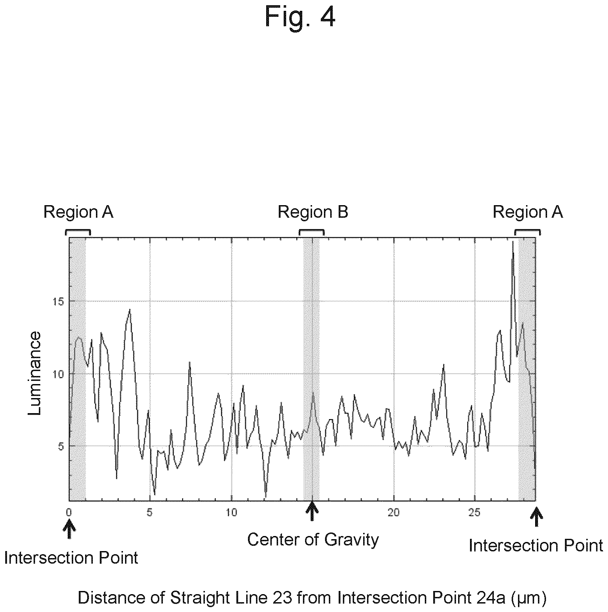

Spectral intensities of $^{25}C_2H^-$ on the cross-sectional surface of the cell adsorption material were each mapped on an image of the cross-sectional surface of the cell adsorption material (an image X). Next, spectral intensities of $^{26}CN^-$ on the cross-sectional surface of the cell adsorption material also were mapped similarly to the image X (an image Y). At this time, in the image X and the image Y, the imaged areas were selected such that the cross-sectional surface image of one cell adsorption material was completely in the visual field of 50 μm×50 μm. Furthermore, each of the image X and the image Y underwent the following measurements using image analysis software, ImageJ. First, as illustrated in Fig. 2, a center of gravity 22 of the cross-sectional surface of the cell adsorption material was measured by surrounding and selecting an outer periphery 21 of the cross-sectional surface of the cell adsorption material. Next, a straight line 23 that passes through the above-described center of gravity 22 and vertically or horizontally crosses the cross-sectional surface of the cell adsorption material was drawn. From two intersection points 24a, 24b between the straight line 23 and the outer periphery 21 of the cross-sectional surface of the cell adsorption material described above, each region within 1.0 μm was determined to be a region A (31) and a region within 0.5 μm radius from the center of gravity of the cross-sectional surface of the cell adsorption material was determined to be a region B (32) (Fig. 3). For each of the regions A and the region B, luminance of the image Y when luminance of the image X was set to be 1 was obtained to be a spectral intensity of $^{26}CN^-$ in the regions A and the region B. Such an example is illustrated in Fig. 4. Total of four straight lines that vertically or horizontally cross the cross-sectional surface of the cell adsorption material were drawn for each 45°, the above-described measurements were performed for each straight line, and the average values of the obtained values were determined to be spectral intensities of $^{26}CN^-$ in the regions A and the region B. Note that, for the average values, values rounded to one decimal place were used.

<Measurement of Modal Pore Radius and Total Volume Ratio>

The modal pore radius is a pore radius having the maximum value of a logarithmic differential pore volume on a diagram on which a logarithmic differential pore volume (dV/d(logr), here, V represents a pore volume per 1 g of the cell adsorption material and r represents a pore radius) is plotted with respect to the pore radius of the cell adsorption material, that is, the modal pore radius is a pore radius having the most frequency. The pore radius and the pore volume were obtained by measuring a degree of freezing-point depression by capillary condensation of water in the pores using a DSC unit (DSC Q100, manufactured by TA Instruments). First, excessive water adhered on a surface was removed from the cell adsorption material in a state of being immersed in distilled water, the cell adsorption material was cooled rapidly to -55°C using the DSC unit, and thereafter, the temperature was increased to 5°C by 0.3°C/minute and a measurement was taken. The obtained peak top temperature of a melting point distribution curve was determined as a melting point, and thus, a melting point depression amount was obtained and the pore radius was calculated by the following Formula (7). Note that, for the pore radius, a value rounded to the nearest whole number was used.

Pore radius (nm) = (33.30 - 0.3181 × melting point depression amount [°C]) / melting point depression amount [°C]    Formula (7)

The pore volume was calculated by the following Formula (8).
[Math. 2]

$$V_{fp} = \int_0^\infty (\frac{dV_{fp}}{dr})\, dR \quad \cdots \quad \text{Formula (8)}$$

In Formula (8), $V_{fp}$ is a pore volume per 1 g of the cell adsorption material, and r is a pore radius.
The total volume of all the pores was obtained by summing volumes of all the pores per 1 g of the cell adsorption material observed in the DSC measurement. The total volume of the pores with a pore radius of 10 nm or more and 250 nm or less was obtained by summing volumes of all the pores with a pore radius of 10 nm or more and 250 nm or less per 1 g of the cell adsorption material observed in the DSC measurement, similarly to the total volume of all the pores. The total volume ratio was calculated by calculating a proportion of the total volume of the pores with a pore radius of 10 nm or more and 250 nm or less with respect to the total volume of all the pores. Note that, for the above-described measurement and calculation method, a reference was made to the description in the document (Kazuhiko Ishikiriyama et al.; JOURNAL OF COLLOID AND INTERFACE SCIENCE, VOL. 171, 103-111 (1995)).

<Measurement of Major Axis of Fibers>

"The major axis of the fibers" was obtained by the following method. 100 samples of a water-insoluble carrier in a fiber form as the cell adsorption material were randomly extracted, and one photograph of a cross-sectional surface (a cross-sectional surface perpendicular to an elongation direction of the fibers) was taken at 3,000-fold magnification for each sample using a scanning electron microscope. Next, image analysis software, ImageJ was used to measure a major axis of each fiber cross-sectional surface. The average value of those values (the average value of the major axes of 100 fiber cross-sectional surfaces in total) was calculated, and a value rounded to the nearest whole number was determined to be the major axis of the fibers.

<Measurement of Roundness>

When the cell adsorption material was a fiber, the measurement was taken by the following method. By using image analysis software, Avizo and ImageJ, on the image Y of the cross-sectional surface of the cell adsorption material as a target circular body obtained in "Measurement of Spectral Intensity of $^{26}CN^-$" described above, the roundness was calculated by the following Formula (9) by surrounding the outer periphery of the cell adsorption material as the target circular body in the image Y and measuring the outer periphery, and a value rounded to two decimal places was used.

$$Roundness = 4\pi \times S/a^2 \dots Formula\ (9)$$

In Formula (9), S is an area of the target circular body, and *a* is a perimeter of the target circular body.

<Measurement of Solidity>

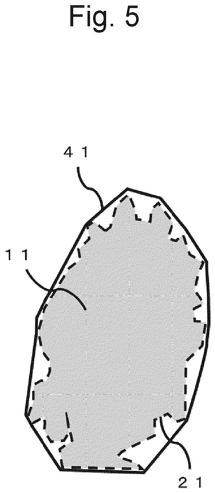

The image Y of the cross-sectional surface of the cell adsorption material obtained in "Measurement of Spectral Intensity of $^{26}CN^-$" described above was analyzed using the image analysis software, Avizo and ImageJ. Such an example is illustrated in Fig. 5. The outer periphery 21 of the cross-sectional surface of the cell adsorption material in the image Y was surrounded, the cross-sectional area of the cell adsorption material and an area of a convex hull 41 with respect to the outer periphery of the cross-sectional surface of the cell adsorption material were obtained, the solidity was calculated by the following Formula (10), and a value rounded to two decimal places was used.

$$Solidity = S_A/S_C \dots Formula\ (10)$$

In Formula (10), $S_A$ is a cross-sectional area of the cell adsorption material, and Sc is an area of the convex hull 41 with respect to the outer periphery of the cross-sectional surface of the cell adsorption material. The convex hull means the smallest convex polygon encompassing all provided points.

<Measurement of Arithmetic Mean Roughness>

Using a shape measuring laser microscope (Color 3D Laser Microscope VK-9700, manufactured by KEYENCE CORPORATION), a surface of the cell adsorption material in a dry state was observed at 100-fold magnification, and a roughness curved line was measured. In the obtained roughness curved line, only the reference length L was extracted in a direction of the average line, the x-axis was set in the direction of the average line of this extracted part and the y-axis was set in a direction of vertical magnification, the roughness curved line was represented as y = f(x), and the arithmetic mean roughness was calculated by the following Formula (3) (compliant with Japanese Industrial Standard B 0601-2001). The reference length L was determined to be 50 $\mu$m, the average value of the values measured at ten different positions was determined to be the arithmetic mean roughness of the surface of the cell adsorption material, and a value rounded to two decimal places was used.

[Math. 3]

$$Ra = \frac{1}{L}\int_0^L |f(x)|\,dx \cdots Formula\ (3)$$

<Measurement of Amino Group Amount>

First, 50 mL of a 6 mol/L sodium hydroxide aqueous solution was added to 1.0 g of the cell adsorption material, and they were stirred for one hour at room temperature. 50 mL of ion exchanged water was added to the above-described cell adsorption material for cleaning, the cleaning was repeated until the above-described ion exchanged water had a pH of 7, and thus, the desalinated cell adsorption material was obtained. The cell adsorption material after the desalination was allowed to stand for 48 hours under reduced pressure at 25°C and was dried. The mass of the adsorption material after the drying was measured, 40 mL of a 0.1 mol/L hydrochloric acid was added, and they were stirred for 30 minutes at room temperature. After the stirring, 5 mL of only the solution remaining after extracting the cell adsorption material (hereinafter referred to as a "dispensing solution 1") was extracted, to which 0.1 mL of a 0.1 mol/L sodium hydroxide aqueous solution was dripped (hereinafter referred to as a "measured solution 1"). They were stirred for ten minutes after the dripping, and then a pH of the measured solution 1 was measured. The ten minute stirring after the dripping and the pH measurement were repeated in a similar way until the pH of the measured solution 1 exceeded 8.5. The dripping amount of the sodium hydroxide aqueous solution when the pH of the measured solution 1 exceeded 8.5 was determined to be a titer per 1 g of the cell adsorption material. Using the titer per 1 g of the cell adsorption material and the following Formula (11), the amino group amount was calculated, and a value rounded to the nearest whole number was used.

$$\text{Amino group amount per 1 g of cell adsorption material } (\mu\text{mol/g}) = V_{HCl\text{-}1}/V_{S1} \times V_F/M_{S1} \times C_{NaOh\text{-}1} \times 1{,}000 \qquad \text{Formula (11)}$$

In Formula (11), $V_{HCl\text{-}1}$ means the amount of the 0.1 mol/L hydrochloric acid added (40 mL), Vsi means the amount of the dispensing solution 1 (5 mL), $V_F$ means the titer (mL), $M_{S1}$ means the mass of the cell adsorption material after the drying (g), and $C_{NaOH\text{-}1}$ means the concentration of the sodium hydroxide aqueous solution (0.1 mol/L).

<Measurement of Amino Group/Amide Group Ratio>

The amide group amount was obtained by the following method. First, 100 mL of a 6 mol/L hydrochloric acid was added to 1.0 g of the dried cell adsorption material, and they were hydrolyzed for 20 hours at 110°C. The cell adsorption material after the hydrolyzing was cleaned with ion exchanged water, and 50 mL of a 6 mol/L sodium hydroxide aqueous solution was added. After the above-described cell adsorption material was inverted and stirred for 30 minutes or more, ion exchanged water was added for cleaning. After repeating the cleaning until the pH of the ion exchanged water added to the above-described cell adsorption material became 7, the above-described cell adsorption material was allowed to stand for 48 hours under reduced pressure at 25°C and dried. Next, the mass of the cell adsorption material after the drying was measured, 30 mL of a 0.1 mol/L hydrochloric acid was added, and they were inverted and stirred for 30 minutes. To the solution remaining after extracting the cell adsorption material (hereinafter referred to as a "dispensing solution 2"), a methyl red solution and a phenolphthalein solution were added (hereinafter referred to as a "measured solution 2"). A 0.02 mol/L sodium hydroxide aqueous solution was dripped to the measured solution, and the time point where the color of the measured solution had turned yellow was determined to be the ending point. The amount of the amide group per 1 g of the cell adsorption material was calculated by the following Formula (12), and a value rounded to the nearest whole number was used.

$$\text{Amino group amount per 1 g of cell adsorption material } (\mu\text{mol/g}) = (V_{S2} \times C_{HCl\text{-}2}/C_{NaOh\text{-}2} - V_{NaOh\text{-}2}) \times C_{NaOH\text{-}2} \times (V_{HCl\text{-}2}/V_{S2})/M_{S2} \times 1{,}000 \qquad \text{Formula (12)}$$

In Formula (12), $V_{S2}$ means the amount of the dispensing solution 2 (mL), $C_{HCl\text{-}2}$ means the concentration of the hydrochloric acid added at the second time (0.1 mol/L), $C_{NaOH\text{-}2}$ means the concentration of the sodium hydroxide aqueous solution dripped to the measured solution 2 (0.02 mol/L), $V_{NaOH\text{-}2}$ means the amount of the sodium hydroxide aqueous solution dripped to the measured solution 2 (mL), $V_{HCl\text{-}2}$ means the amount of the hydrochloric acid added at the second time (mL), and $M_{S2}$ means the mass of the cell adsorption material after the drying (g).

Using the amino group amount calculated in "Measurement of Amino Group Amount" described above, the amino group/amide group ratio was calculated, and a value rounded to three decimal places was used.

<Adsorption Test of LAP Positive Immune Cells>

The adsorption rate of LAP positive immune cells in the cell adsorption material was measured by an adsorption column liquid passage type immune cell adsorption test using human blood. A flow cytometry (FACSLyric, manufactured by Becton, Dickinson and Company) was used for an analysis.

First, a filled portion (diameter 10 mm, height 14 mm) of the cell adsorption column was filled with 0.3 g in dry mass of the cell adsorption material and normal saline, and high-pressure steam sterilization (117°C, 105 minutes) was performed. Thereafter, a heparinized saline solution with a final concentration of 50 unit/mL was passed in the cell adsorption column for 2.5 minutes at 1.54 mL/minute, and a priming treatment was performed. Next, blood (to which heparin with a final concentration of 5 unit/mL was added) drawn from a healthy human was passed at 0.31 mL/ minute, and blood was extracted at the column outlet after ten minutes. Blood at the column inlet side was also extracted after ten minutes. 100 $\mu$L of the extracted blood was each dispensed in a tube (manufactured by FALCON) made of polypropylene with a capacity of 5 mL, and immune cells were stained by adding various kinds of fluorescent labeled antibodies. For various kinds of the fluorescent labeled antibodies, fluorescein isothiocyanate (hereinafter referred to as "FITC") labeled anti-human CD4 antibody (manufactured by BioLegend, Inc.), phycoerythrin (hereinafter referred to as "PE") labeled anti-human CD42b antibody (manufactured by BioLegend, Inc.), and allophycocyanin (hereinafter referred to as "APC") labeled anti-human LAP antibody (manufactured by R&D Systems, Inc.) were used. A flow cytometer was used for detecting the number of LAP positive immune cells. For LAP positive T-cell, a CD4 positive and LAP positive cell was an evaluation target, and for LAP positive platelet, a CD42b positive and LAP positive cell was an evaluation target.

The adsorption rate of LAP positive T-cells was calculated by the following Formula (13), and a value rounded to the nearest whole number was used.

Adsorption rate of LAP positive T-cells (%) = (1 - number of LAP positive T-cells in blood with addition of cell adsorption material/number of LAP positive T-cells in blood without addition of cell adsorption material) $\times$ 100        Formula (13)

The adsorption rate of LAP positive platelets was calculated by the following Formula (14).

Adsorption rate of LAP positive T-cells (%) = (1 - number of LAP positive platelets in blood with addition of cell adsorption material/number of LAP positive platelets in blood without addition of cell adsorption material) $\times$ 100       Formula (14)

<Manufacturing Raw Knitted Fabric (Water-Insoluble Carrier) and Intermediate (Linker Bound Water-Insoluble Carrier)>

As a water-insoluble carrier, a sea-island composite fiber (diameter of fiber: 20 $\mu$m) was spun. The sea-island composite fiber had 264 islands of island component made of polypropylene (Prime Polymer Co., Ltd.; J105WT) and a sea component made of 90 mass% of polystyrene (weight average molecular weight: 181,000) and 10 mass% of polypropylene (Prime Polymer Co., Ltd.; J105WT), while a ratio of the island to the sea (mass ratio) was 50:50. The obtained 36 fibers were combined to form a knitted fabric (hereinafter, referred to as a "raw knitted fabric"). It should be noted that arithmetic mean roughness of a fiber surface is affected by the number of islands, a sea/island ratio, molecular weights of polystyrene and polypropylene, and the like.

Paraformaldehyde (hereinafter, referred to as "PFA," 2 g) was dissolved in a mixed solution of nitrobenzene (20 mL) and sulfuric acid (13 mL) at 10°C (hereinafter referred to as a "PFA solution"). Furthermore, NMCA (47 g) was dissolved in a mixed solution of nitrobenzene (259 mL) and sulfuric acid (169 mL) at 10°C (hereinafter referred to as an "NMCA solution"). After immersing the raw knitted fabric (10 g) in the PFA solution, the NMCA solution was promptly added and stirred. After immersion and stirring for one hour, the knitted fabric was taken out. After cleaning with excessive nitrobenzene, the knitted fabric was displaced and cleaned with methanol, and further cleaned with ion exchanged water, to obtain an $\alpha$-chloracetamidomethylated knitted fabric (hereinafter referred to as an "intermediate"). A series of operation from the preparation of the PFA solution to the cleaning of the knitted fabric using the methanol was performed at 15°C or less.

[Example 1]

DETA (23.3 mL) was dissolved in ion exchanged water (405.0 mL) to prepare a 500 mmol/L DETA aqueous solution. The intermediate (10 g) was immersed in this DETA aqueous solution, and they were stirred for three hours at 40°C. Thereafter, the intermediate was immersed and cleaned in methanol, ion exchanged water, was dried, and a cell adsorption material E1 was obtained.

[Example 2]

After ion exchanged water (364.8 mL) and dimethylsulfoxide (hereinafter referred to as "DMSO," 63.8 mL) were mixed, DETA (23.3 mL) was added to prepare a 500 mmol/L DETA-10 mass% DMSO aqueous solution. The intermediate (10 g) was immersed in this DETA-10 mass% DMSO aqueous solution, and they were stirred for three hours at 40°C. The intermediate was cleaned and dried similarly to Example 1, and a cell adsorption material E2 was obtained.

[Example 3]

DETA (0.9 mL) was dissolved in ion exchanged water (427.6 mL) to prepare a 20 mmol/L DETA aqueous solution. The intermediate (10 g) was immersed in this 20 mmol/L DETA aqueous solution, and they were stirred for three hours at 40°C. The intermediate was cleaned and dried similarly to Example 1, and a cell adsorption material E3 was obtained.

[Comparative Example 1]

The intermediate (10 g) was immersed in ion exchanged water (428.6 mL), and they were stirred for three hours at 40°C. The intermediate was cleaned and dried similarly to Example 1, and a cell adsorption material C1 was obtained.

[Comparative Example 2]

After adding triethylamine (28.6 mL) in DMSO (398.4 mL), DETA (0.046 mL) was added to prepare a 1 mmol/L DETA-DMSO solution. The intermediate (10 g) was immersed in this DETA-DMSO solution, and they were stirred for three hours at 40°C. Thereafter, the intermediate was immersed and cleaned in DMSO, methanol, and then, water, was dried, and a cell adsorption material C2 was obtained.

For the cell adsorption materials (E1, E2, E3, C1, C2), a spectral intensity of $^{26}CN^-$, a modal pore radius, a total volume of the pores with a pore radius of 10 nm or more and 250 nm or less, roundness, solidity, a major axis, arithmetic mean roughness of the surface, an amino group amount, an amino group/amide group ratio, and adsorption rates of LAP positive T-cells and LAP positive platelets were measured by the above-described methods. The results are illustrated in Table 1.

[Table 1]

| | | Example 1 | Example 2 | Example 3 | Comparative Example 1 | Comparative Example 2 |
|---|---|---|---|---|---|---|
| Water-Insoluble Carrier | Nitrogen-Containing Compound Concentration (mmol/L) | 500 | 500 | 20 | 0 | 1 |
| | Reaction Solvent | Water | 10% DMSO/ Water | Water | Water | DMSO |
| | Major Axis of Fiber ($\mu$m) | 20 | 20 | 20 | 20 | 20 |
| | Arithmetic Mean Roughness of Surface ($\mu$m) | 0.46 | 0.31 | 0.47 | 0.38 | 0.50 |
| | Roundness of Cross-Sectional Surface | 0.83 | 0.75 | 0.80 | 0.80 | 0.52 |
| | Convexity of Cross-Sectional Surface | 0.97 | 0.93 | 0.96 | 0.96 | 0.87 |
| | Spectral Intensity of $^{26}CN^{-}$ in Region A | 1.5 | 1.2 | 1.6 | 0.4 | 1.3 |
| | Spectral Intensity of $^{26}CN^{-}$ in Region A/Spectral Intensity of $^{26}CN^{-}$ in Region B | 7.0 | 6.2 | 3.7 | 5.1 | 1.4 |
| | Modal Pore Radius (nm) | 68 | 67 | 54 | 65 | 4 |
| | Proportion Occupied by Total Volume of Pores with Pore Radius of 10 nm or more and 250 nm or less (%) | 100 | 100 | 100 | 100 | 35 |
| | Amino Group Amount ($\mu$mol/g) | 65 | 35 | 62 | 0 | 300 |
| | Amino Group/Amide Group Ratio | 0.023 | 0.012 | 0.021 | 0.000 | 0.103 |
| Blood Cell Removability | Adsorption Rate of LAP Positive T-Cell (%) | 86 | 82 | 84 | 27 | 51 |
| | Adsorption Rate of LAP Positive Blood Platelet (%) | 96 | 94 | 94 | 45 | 59 |

Abbreviations in the table are as follows.

DETA: diethylene triamine

DMSO: dimethylsulfoxide

Industrial Applicability

The cell adsorption material and the cell adsorption column of the present invention can adsorb cells, such as LAP positive immune cells, with high efficiency. Therefore, an application to cancer therapy is expected. The cell adsorption material and the cell adsorption column of the present invention can be used in combination with the cell infusion treatment that activates dendritic cells, natural killer cells, and the like.

Reference Signs List

| | |
|---|---|
| 1 | Container body |
| 2 | Inflow port |
| 3 | Outflow port |
| 4 | Filter |
| 5 | Partition plate |
| 5a | Opening of partition plate |
| 6 | Filter |
| 7 | Partition plate |
| 7a | Support protrusion of partition plate |
| 7b | Through hole of partition plate |
| 8 | Pipe |
| 9 | Flow passage |
| 10 | Through-hole |
| 11 | Cell adsorption material |
| Q | Blood flow |
| 21 | Outer periphery of cross-sectional surface of cell adsorption material |
| 22 | Center of gravity of cross-sectional surface of cell adsorption material |
| 23 | Straight line passing through center of gravity 22 of cross-sectional surface of cell adsorption material and transversing cross-sectional surface vertically or horizontally |
| 24a, 24b | Intersection point between straight line 23 and outer periphery 21 of cross-sectional surface of cell adsorption material |
| 31 | Region A |
| 32 | Region B |
| 41 | Convex hull with respect to outer periphery of cross-sectional surface of cell adsorption material |

## Claims

1. A cell adsorption material, comprising fibers or particles of a water-insoluble carrier to which at least one nitrogen-containing compound is bound, the nitrogen-containing compound being selected from the group consisting of polyamines and aliphatic amines,

   wherein the fibers or the particles have a region A and a region B, the region A being a region within 1.0 $\mu$m from an outermost surface of a cross-sectional surface of the fibers or the particles, the region B being a region within 0.5 $\mu$m radius from a center of gravity of the cross-sectional surface of the fibers or the particles, a spectral intensity of $^{26}CN^-$ satisfies formulae (1) and (2) below when a spectral intensity of $^{25}C_2H^-$ in each of the regions measured by Time of Flight Secondary Ion Mass Spectrometry is set to be 1:

$$0.5 \leq \text{the spectral intensity of } {}^{26}\text{CN}^- \text{ in the region A} \leq 3.0 \ldots \text{Formula (1)};$$

$$3.0 \leq \text{the spectral intensity of } {}^{26}\text{CN}^- \text{ in the region A/the spectral intensity of } {}^{26}\text{CN}^- \text{ in the region B} \leq 20.0 \quad \text{Formula (2)}.$$

2. The cell adsorption material according to claim 1,

   wherein the fibers or the particles have a modal pore radius of 10 nm or more and 100 nm or less, and
   a total volume of pores with a pore radius of 10 nm or more and 250 nm or less with respect to a total volume of all the pores in the fibers or the particles is 50% or more and 100% or less.

3. The cell adsorption material according to claim 1 or 2,
   wherein the cross-sectional surface of the fibers or the particles has a roundness of 0.75 or more and 0.95 or less.

4. The cell adsorption material according to claim 1 or claim 2,
   wherein the fibers or the particles have a major axis of 15 μm or more and 50 μm or less.

5. The cell adsorption material according to claim 1 or 2,
   wherein the fibers or the particles have an arithmetic mean roughness of a surface of 0.01 μm or more and 1.00 μm or less.

6. The cell adsorption material according to claim 1 or 2,

   wherein the nitrogen-containing compound is a polyamine represented by General Formula (I) below:

   $$R^1R^2N\text{-}X\text{-}NR^3R^4 \ldots \qquad (I)$$

   in General Formula (I), X is a saturated or unsaturated aliphatic hydrocarbon group having 2 to 20 carbon atoms, or a heteroatom-containing carbon chain in which 1 to 5 carbon atoms of a saturated or unsaturated aliphatic hydrocarbon group having 3 to 20 carbon atoms are replaced with a nitrogen atom, a hydrogen atom that bonds to the nitrogen atom may be replaced with an alkyl group or an alkyl group having an amino group, and $R^1$ to $R^4$ are each independently a hydrogen atom or an alkyl group.

7. The cell adsorption material according to claim 1 or 2,
   wherein the nitrogen-containing compound is bound to the water-insoluble carrier via a linker.

8. The cell adsorption material according to claim 1 or 2,
   wherein the cell is an immune cell.

9. The cell adsorption material according to claim 8,
   wherein the immune cell is a LAP positive immune cell.

10. The cell adsorption material according to claim 9,
    wherein the LAP positive immune cell is a LAP positive T-cell or a LAP positive platelet.

11. A cell adsorption column comprising the cell adsorption material according to claim 1 or 2.

12. The cell adsorption column according to claim 11 for use in blood purification therapy.

# Fig. 1

# Fig. 2

# Fig. 3

# Fig. 4

Distance of Straight Line 23 from Intersection Point 24a (μm)

Fig. 5

## INTERNATIONAL SEARCH REPORT

| International application No. |
|---|
| **PCT/JP2022/039889** |

**A. CLASSIFICATION OF SUBJECT MATTER**

*A61M 1/36*(2006.01)i; *B01D 15/00*(2006.01)i; *B01J 20/22*(2006.01)i; *B01J 20/26*(2006.01)i; *B01J 20/28*(2006.01)i
FI: A61M1/36 165; B01J20/22 C; B01J20/28 Z; B01D15/00 101B; B01D15/00 M; B01J20/26 H

According to International Patent Classification (IPC) or to both national classification and IPC

**B. FIELDS SEARCHED**

Minimum documentation searched (classification system followed by classification symbols)

A61M1/36; B01D15/00; B01J20/22; B01J20/26; B01J20/28

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2019/049961 A1 (TORAY INDUSTRIES, INC.) 14 March 2019 (2019-03-14) paragraphs [0004]-[0007], [0018]-[0044], [0106]-[0116] | 1-12 |
| A | WO 2019/049962 A1 (TORAY INDUSTRIES, INC.) 14 March 2019 (2019-03-14) entire text, all drawings | 1-12 |
| A | JP 2021-137244 A (ASAHI KASEI MEDICAL CO., LTD.) 16 September 2021 (2021-09-16) entire text, all drawings | 1-12 |
| A | JP 2010-201345 A (TERAMOTO, Kazuo) 16 September 2010 (2010-09-16) entire text, all drawings | 1-12 |
| A | JP 2006-288571 A (TORAY INDUSTRIES, INC.) 26 October 2006 (2006-10-26) entire text, all drawings | 1-12 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 December 2022** | **10 January 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

| Patent document cited in search report | | | Publication date (day/month/year) | Patent family member(s) | | Publication date (day/month/year) |
|---|---|---|---|---|---|---|
| WO | 2019/049961 | A1 | 14 March 2019 | US 2020/0246776 A1 paragraphs [0004]-[0007], [0022]-[0061], [0117]-[0131] EP 3679966 A1 CN 111065425 A KR 10-2020-0051586 A | | |
| WO | 2019/049962 | A1 | 14 March 2019 | US 2020/0215253 A1 entire text, all drawings EP 3679964 A1 KR 10-2020-0051578 A CN 111278483 A | | |
| JP | 2021-137244 | A | 16 September 2021 | (Family: none) | | |
| JP | 2010-201345 | A | 16 September 2010 | (Family: none) | | |
| JP | 2006-288571 | A | 26 October 2006 | US 2009/0275874 A1 entire text, all drawings WO 2006/106972 A1 EP 1886704 A1 CA 2603073 A1 CN 101151056 A KR 10-2007-0116667 A | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60193468 A
- WO 2008038785 A

- WO 2019049962 A

**Non-patent literature cited in the description**

- **KAZUHIKO ISHIKIRIYAMA et al.** *JOURNAL OF COLLOID AND INTERFACE SCIENCE,* 1995, vol. 171, 103-111